# EUROPÄISCHE PATENTANMELDUNG

(11) **EP 4 260 710 A1**
(43) Veröffentlichungstag der Anmeldung: **18.10.2023**
(21) Anmeldenummer: 22168384.0
(22) Anmeldetag: 14.04.2022
(51) Int. Cl.: A23J 1/18, A23J 3/20, A23L 31/00, C12P 21/00, C12N 1/14, C12N 1/22

(54) **VERFAHREN ZUM HERSTELLEN VON PROTEINEN AUF DER BASIS VON STOFFFRAKTIONEN AUS DER NAHRUNGSMITTELINDUSTRIE**

(71) Anmelder: Kontor N GmbH & Co. KG, 18439 Stralsund (DE)
(72) Erfinder: NORDMANN, Juergen, 18573 Rambin (DE); REINEKE-LAUTENBACHER, Jens, 18439 Stralsund (DE); NORDMANN, Jan Malte, 18573 Rambin (DE)
(74) Vertreter: Hauck Patentanwaltspartnerschaft mbB

(57) **Zusammenfassung**

Verfahren zum Herstellen von Proteinen umfassend die folgenden Schritte:
• Bereitstellen eines Nährmediums enthaltend einen Treber aus der Herstellung von Bier oder einem anderen Getränk auf der Basis von Getreide,
• Zusammenbringen des Nährmediums mit mindestens einem Pilz aus der Abteilung der Ständerpilze (Basidiomyceten), der imstande ist, auf dem Medium eine Mischung von Proteinen zu bilden,
• Umsetzen des Trebers zu der Mischung von Proteinen mithilfe des Pilzes.

## Beschreibung

Die Erfindung betrifft ein Verfahren zum Herstellen von Proteinen auf der Basis einer Stofffraktion aus der Nahrungsmittelindustrie.

Beim Brauen von Bier wird zunächst aus Gerste oder anderem Getreide Malz hergestellt. Durch Mälzen werden Enzyme freigesetzt, die aus der Stärke des Korns Malzzucker für die spätere Vergärung erzeugen. Hierzu ist der Maischprozess erforderlich. Beim Maischen werden das vermälzte und geschrotete Getreide mit Brauwasser unter Rühren vermischt und erhitzt, die Stärke durch die Enzyme in Malzzucker (Malthose) umgewandelt und die Malinhaltsstoffe als Extrakt gewonnen. Die flüssige Bierwürze wird durch Abläutern im Läuterbottich oder Maischefilter bei Temperaturen von 72° bis 80°C von den festen Bestandteilen der Maische getrennt, dem sogenannten Biertreber. Das Hauptprodukt für die Bierherstellung ist die Bierwürze. Diese wird gekocht und mit Hopfen versetzt, sodass sich dessen Aromastoffe lösen. Das Kochen bewirkt auch, dass die Bierwürze keimfrei wird. Nach Klärung der Bierwürze wird durch Abkühlen und Zusatz von Hefe der Gärprozess eingeleitet. Bei der Gärung wird Malzzucker in Alkohol und Kohlensäure umgewandelt. Die weitere Behandlung des bei der Bierherstellung in großen Mengen anfallenden Trebers stellt vor allem für kleine und mittelständische Brauereien ein Problem dar. Der aus dem Läuterbottich entnommene Biertreber weist eine kurze mikrobiologische Haltbarkeit von nur wenigen Tagen auf. Der Biertreber wird als Futtermittel in der Milchviehwirtschaft und der Rindermast, als Dünger, für die Strom- und Wärmegewinnung mittels Biogasanlagen und in getrocknetem Zustand für die Wärmeerzeugung durch Verbrennung in Kombination mit Holzhackschnitzeln genutzt.

Bereits bekannt ist die Produktion von Proteinen für die menschliche Ernährung mithilfe von Pilzen. So wurden beispielsweise Hefen für die Proteinherstellung eingesetzt. Unter dem Handelsnamen Quorn^{™} wird ein aus dem fermentierten Mycel des Schimmelpilzes (Ascomyceten) *Fusarium venenatum* hergestelltes Fleischersatzprodukt vermarktet.

In Indonesien wird durch Beimpfung gekochter Sojabohnen mit verschiedenen Rhizopus-Arten das traditionelle Fermentationsprodukt "Tempeh" erzeugt. Bei den Pilzen handelt es sich um Schimmelpilze aus der Abteilung der Jochpilze. Die fermentierte Masse wird in Stücke geschnitten, gebraten und verzehrt. Durch die Fermentation werden die Proteine der Sojabohnen aufgewertet und verdauungsschädliche Bestandteile abgebaut.

Seit einigen Jahren gibt es Bestrebungen, klassische Speisepilze aus der Klasse der Basidiomyceten für die Produktion von Proteinen und/oder Aromastoffen einzusetzen, da diese einen hohen Proteinanteil aufweisen, vielfältige Aromen ausbilden und das Pilzprotein eine hohe biologische Wertigkeit aufweist.

In einem Fachartikel ist die Herstellung von Proteinen durch Fermentation von Nebenströmen aus der Nahrungsmittelindustrie mittels Basidiomyceten beschrieben (Zorn, H. et al. Upcycling of Food Industry Side Streams by Basidiomycetes for Production of a Vegan Proteine Source, International Journal of Recycling of Organic Waste in Agriculture 2019, 8:447 - 445). Der Nährwert von Apfeltrester wurde durch Fermentation mit dem Pilz *Pleurotus sapidus* stark erhöht und die resultierende Biomasse wurde als geeignete alternative Proteinquelle angesehen. Der Aminosäureanteil wurde von ungefähr 5% beim Apfeltrester auf 24% beim fermentierten Apfeltrester erhöht und der fermentierte Apfeltrester wies eine hohe biologische Wertigkeit von 86 auf und hierdurch wird ein guter Nährwert für Menschen indiziert.

In einem weiteren Fachartikel (Zorn, H. et al. Characterization of the Nutritional Composition of a Biotechnologically Produced Oyster Mushroom and its Physiological Effects in Obese Zucker Rats, Mol. Nutr. Food Res. 2020, 64) sind physiologische (antisteatotische und entzündungshemmende) Effekte einer Nährstoffzusammensetzung eines biotechnologisch produzierten Austernpilzes bei Untersuchungen am Rattenmodell beschrieben.

In der WO 2013/034613 A2 ist ein Verfahren zur Herstellung eines Getränks oder einer Getränkebase beschrieben, bei dem in wenigstens einem Fermentationsprozess ein Medium fermentiert wird, das pumpbar ist, und bei dem der Fermentationprozess aerob durchgeführt wird, wobei das Medium durch Mycel von wenigstens einem Basidiomyceten fermentiert wird. Bei einem Ausführungsbeispiel wird ungehopfte Bierwürze mit dem Mycel der Basidiomyceten *Ischnoderma benzoinum, Tyromyces chioneus* und *Wolfiporia cocos* fermentiert. Das Mycel des Basidiomyceten wird durch Zentrifugieren abgetrennt und die restliche Probe sensorisch beurteilt. Es wird festgestellt, dass nach aerober Fermentation von ungehopfter Bierwürze ansprechend riechende und gut schmeckende Getränke erhalten werden, die sich deutlich von nicht fermentierter Bierwürze unterscheiden. Diese Verwendung der Bierwürze für die Getränkeherstellung konkurriert mit ihrer Verwendung für die Bierherstellung.

Davon ausgehend liegt der Erfindung die Aufgabe zugrunde, ein Verfahren zum Herstellen von Proteinen mit hoher biologischer Wertigkeit zu schaffen, das als Nährmedium eine Stofffraktion aus der Nahrungsmittelindustrie verwendet.

Das erfindungsgemäße Verfahren zum Herstellen von Proteinen umfasst die folgenden Schritte:
- Bereitstellen eines Nährmediums (Umsetzungsmediums) enthaltend einen Treber aus der Herstellung von Bier oder eines anderen Getränkes auf der Basis von Getreide,
- Zusammenbringen des Nährmediums mit mindestens einem Pilz aus der Abteilung der Ständerpilze (Basidiomyceten), der imstande ist, auf dem Umsetzungsmedium eine Mischung von Proteinen zu bilden,
- Umsetzung des Trebers zu der Mischung von Proteinen mithilfe des Pilzes.

Mittels des erfindungsgemäßen Verfahrens wird erstmals eine Stofffraktion mit hohem Feststoffgehalt aus der Maische eines Brauprozesses oder eines anderen Verfahrens zum Herstellen von Getränken auf der Basis von Getreide für die Herstellung eines protein- und/oder aromastoffhaltigen Produktes mit Hilfe von Pilzen aus der Klasse der Basidiomyceten herangezogen. Bei den herkömmlichen Verfahren zum Herstellen von Bier oder anderen Getränken über eine Maische aus vermälztem und/oder unvermälztem Getreide wird das Getreide nur teilweise zu für den Menschen verwertbaren Stoffen verarbeitet. So bauen die beim Bierbrauen verwendeten Hefen das vermälzte Getreide nur teilweise in für den Menschen verdaubare Stoffe um. Die Erfindung macht sich zu Nutze, dass die beim Bierbrauen nicht von den Hefen verwertbaren Bestandteile wie Zellulose und Hemizellulose mit Hilfe von Basidiomyceten zu vom Menschen verdaubaren Proteinen umgewandelt werden können. Dabei ist von Vorteil, dass die dem Verfahren zugeführten Ausgangsstoffe von einer für die Herstellung von Bier oder anderen Getränken geeigneten Qualität sind, sodass sie sich grundsätzlich auch für die Herstellung von Lebensmitteln und von anderen vom Menschen zu konsumierenden Produkten eignen. Bei dem Verfahren wird die Maische in eine erste Stofffraktion mit geringem Feststoffgehalt (z.B. Bierwürze) und in eine zweite Stofffraktion mit hohem Feststoffgehalt (z.B. Biertreber) fraktioniert, die erste Stofffraktion für die Herstellung von Bier oder anderen Getränken und die zweite Stofffraktion für die Herstellung von proteinreichem Pilzmycel eingesetzt. Bei der herkömmlichen Bierherstellung wird die beim Abläutern der Bierwürze anfallende Stofffraktion mit hohem Feststoffgehalt als Biertreber in großen Silos zwischengelagert und überwiegend zur Verwendung als Tierfutter an die Agrarwirtschaft abgegeben. Die Stofffraktion mit hohem Feststoffgehalt ist jedoch aufgrund ihrer Herstellung unter Verwendung von für die Erzeugung von Nahrungsmitteln geeigneten Einsatzstoffen, ihrer stofflichen Zusammensetzung, ihres großen Mengenanteils und ihrer einheitlichen Zusammensetzung und grundsätzlich hohen Qualität besonders für die Herstellung von Proteinen für die Aufnahme durch den Menschen geeignet.

Basidiomyceten (Ständerpilze) umfassen die für den Menschen zum Verzehr geeigneten essbaren Ständerpilze. Sie verfügen über ein sehr breites biochemisches Transformationspotential und unterscheiden sich hierdurch von niederen Pilzen und Bakterien. Erfindungsgemäß wird dieses Potential dafür genutzt, Proteine mit einer hohen biologischen Wertigkeit zur Verfügung zu stellen.

Basidiomyceten sind in der Lage, proteinreiche Pilzmycele zu bilden, deren Proteine eine hohe biologische Wertigkeit aufweisen können. Untersuchungen im Rahmen der Erfindung haben gezeigt, dass mittels Basidiomyceten fermentierter Treber eine besonders hohe biologische Wertigkeit von über 90 aufweist und deshalb besonders gut vom Menschen verwertet werden kann. Die biologische Wertigkeit der Pilzproteine ist mit der von Rindfleisch vergleichbar und weitaus größer als bei pflanzlichen Proteinen oder fermentiertem Apfeltrester.

Zudem können die Fermentationsprodukte von Basidiomyceten Aromastoffe mit vielfältigen Geschmacks- und/oder Geruchsaromen enthalten, beispielsweise Aromastoffe z.B. mit Frucht-, Beeren-, Kräuter-, Gewürz-, fleischigen und/oder fischigen Aromanoten. Untersuchungen im Rahmen der Erfindung haben gezeigt, dass bei der Fermentation von Treber mittels Basidiomyceten besonders ansprechende Aromastoffe erzeugt werden.

Durch das Verfahren werden wertvolle Rohstoffe für die Herstellung von Bier oder anderen Getränken auf der Basis von Getreide zusätzlich für die Herstellung von ernährungsphysiologisch, gustatorisch und olfaktorisch besonders wirksamen und interessanten Produkten genutzt. Neben den vorgenannten vorteilhaften Effekten können mittels Basidiomyceten hergestellte protein- und/oder aromastoffhaltige Pilzmycele entzündungshemmende oder andere gesundheitsfördernde Wirkungen haben.

Untersuchungen im Rahmen der Erfindung haben ergeben, dass der Glutengehalt des mittels Basidiomyceten fermentierten Trebers stark gegenüber dem Glutengehalt des Trebers abgebaut wurde. Aufgrund von Untersuchungen über das Ausmaß der Biokonversion verschiedener Substrate durch Pilze aus der Klasse der Basidiomyceten ist zu erwarten, dass Basidiomyceten generell die Eigenschaft haben, in einem Nährsubstrat enthaltenes Gluten in erheblichem Ausmaß abzubauen.

Der CO₂-Fußabdruck der Proteinherstellung ist weitaus kleiner als bei der Fleischerzeugung. Die Ökobilanz des Gesamtverfahrens ist besser als die Gesamtbilanz der herkömmlichen Herstellung von Bier oder anderer Getränke auf der Basis von Getreide und der Herstellung von bekömmlichen Produkten, die von den protein- und/oder aromastoffhaltigen Produkten substituiert werden.

Gemäß einer Ausführungsart ist der Ständerpilz ausgewählt aus der nachfolgenden Gruppe von Pilzen: *Pleurotus eryngii, Pholiota nameko, Cyclocybe aegerita.*

Mit diesen Basidiomyceten können Proteinmischungen mit hoher biologischer Wertigkeit, attraktiven Aromaprofilen und geringen Glutenanteilen erreicht werden.

Gemäß einer weiteren Ausführungsart weist die Mischung von Proteinen eine biologische Wertigkeit von mindestens 94, vorzugsweise mindestens 97 auf.

Gemäß einer weiteren Ausführungsart enthält die Mischung von Proteinen mindestens einen Aromastoff oder Mischung von Aromastoffen, der mindestens eine Verbindung enthält, die aus der Stoffklasse der Terpene (z.B. Linalool, Linaloooxid, (*E*)-Nerolidol), der Carbonyle (z.B. 1-Octen-3-on, Zimtaldehyd), der Lactone (z.B. gamma-Nonalacton) und der Alkohole (z.B. 1-Hexanol, 2-Methyl-3-buten-2-ol) ausgewählt ist.

Gemäß einer weiteren Ausführungsart ist der Ständerpilz ausgewählt aus einer Gruppe von Ständerpilzen mit der Eigenschaft, den Glutenanteil des Nährmediums um mindestens 90% abzubauen.

Gemäß einer weiteren Ausführungsart weist die Mischung von Proteinen einen Gliadingehalt von maximal 0,5 g/100 g auf. Messungen haben ergeben, dass der Gliadingehalt des Trebers durch die Fermentation stark abgebaut wurde. Erreicht wurde ein Glutenabbau von über 98% bezogen auf die Trockenmasse. Durch Verarbeitung der Proteinmischung in einer Lebensmittelzusammensetzung ist es möglich, den Grenzwert für Glutenfreiheit von 20 mg/kg zu unterschreiten. Es ist zu erwarten, dass durch Optimierung des Herstellungsverfahrens der Glutenabbau noch gesteigert werden kann.

Gemäß einer weiteren Ausführungsart besteht das Medium aus Treber oder im Wesentlichen aus Treber. In der Regel weist unter den für die Herstellung von Proteinen und/oder Aromastoffen geeigneten Stofffraktionen bei der Herstellung von Bier oder anderen Getränken auf der Basis von Getreide die beim Maischen anfallende Stofffraktion mit dem hohen Feststoffgehalt (Treber) den größten Anteil auf, sodass ihre Biokonversion zu protein- und/oder aromastoffhaltigem Pilzmycel besonders effizient ist. Für die Umsetzung zu einer Proteinmischung können aber auch weitere Stofffraktionen aus der Getränkeherstellung zusätzlich verwendet werden.

Gemäß einer weiteren Ausführungsart umfasst das Nährmedium neben dem Biertreber einen weiteren Nebenstrom aus einem Verfahren der Herstellung von Bier oder eines anderen Getränks. Gemäß einer weiteren Ausführungsart wird zusätzlich vermälztes und/oder unvermälztes Getreide aus dem Mälzereiprozess für die Proteinherstellung eingesetzt. Beispielsweise können Überschussmengen von vermälztem und/oder unvermälztem Getreide eingesetzt werden.

Gemäß einer weiteren Ausführungsart wird für die Herstellung der Proteinmischung zusätzlich mindestens eine der Stofffraktionen Bierhefe, Prozessbier, Schlempe oder Prozesswasser eingesetzt. Die hierfür eingesetzte Bierhefe ist beispielsweise überschüssige Hefe oder Althefe und das hierfür eingesetzte Prozessbier ist beispielsweise verdünnte Würze oder verdünntes Bier.

Gemäß einer weiteren Ausführungsart wird die Umsetzung in einer Submers-Kultur durchgeführt. Hierbei wird die Fermentation innerhalb der Dispersion aus der eingesetzten Stofffraktion und dem Inokulat in der wässrigen Phase durchgeführt. Diese Submers-Fermentation ist vorteilhaft für die Kombination mit einem Brauprozess, weil dieser im industriellen Maßstab überwiegend mit flüssigen bzw. pumpfähigen Medien in Misch-, Reaktions- und Lagerbehältern und diese verbindenden Rohrleitungen, in Pumpen und sonstigen Fördereinrichtungen durchgeführt wird. Alternativ wird das inokulierte Nährmedium emers fermentiert.

Gemäß einer weiteren Ausführungsart wird das Nährmedium vor der Umsetzung mit Wasser verdünnt, vorzugsweise mit Prozesswasser aus der Herstellung des Getränks. Hierdurch wird die Pumpfähigkeit des Nährmediums verbessert und das Prozesswasser kann einer Verwertung zugeführt werden.

Gemäß einer weiteren Ausführungsart wird das Proteine und/oder Aromastoffe enthaltende Pilzmycel als Endprodukt verwendet, beispielsweise als Lebensmittel oder Nutraceutical, d.h. als Lebensmittel mit pharmazeutischem Zusatznutzen.

Gemäß einer weiteren Ausführungsart werden die Proteine zumindest teilweise vom Pilzmycel abgetrennt, vorzugsweise durch Extraktion. Die abgetrennten Stoffe werden beispielsweise direkt als Endprodukt verwendet oder mit anderen Stoffen zu Endprodukten verarbeitet.

Ferner betrifft die Erfindung eine proteinreiche Zusammensetzung herstellbar nach dem Verfahren eines der vorhergehenden Ansprüche. Der Zusammensetzung kommen die zu dem Verfahren angegebenen Vorteile entsprechend zu. Dies gilt auch für die nachfolgenden Ausführungsarten der Zusammensetzung.

Gemäß einer weiteren Ausführungsart weist die Zusammensetzung eine biologische Wertigkeit von mindestens 94, vorzugsweise von mindestens 97 auf.

Gemäß einer weiteren Ausführungsart umfasst die Zusammensetzung einen Aromastoff oder eine Mischung von Aromastoffen, die zumindest eine Verbindung enthält, die aus der Stoffklasse derTerpene (z.B. Linalool, Linaloooxid, (*E*)-Nerolidol), der Carbonyle (z.B. 1-Octen-3-on, Zimtaldehyd), der Lactone (z.B. gamma-Nonalacton) und der Alkohole (z.B. 1-Hexanol, 2-Methyl-3-buten-2-ol) ausgewählt ist ausgewählt ist.

Gemäß einer weiteren Ausführungsart enthält die Zusammensetzung maximal 0,5 g/100 g Gliadin.

Ferner betrifft die Erfindung eine der Ernährung, dem Genuss oder der Gesundheitsförderung dienende Zubereitung umfassend oder bestehend aus einer Zusammensetzung einer der vorbeschriebenen Arten. Der Zubereitung kommen die für die Zusammensetzung beschriebenen vorteilhaften Wirkungen entsprechend zu. Die biologische Wertigkeit, die Aromastoffe und der Gliadingehalt der Zubereitung setzen sich aus den Werten der Zusammensetzung und gegebenenfalls weiterer Komponenten der Zubereitung zusammen.

Ferner betrifft die Erfindung die Verwendung einer Zusammensetzung als eine der vorbeschriebenen Arten in einer der Ernährung, dem Genuss oder der Gesundheitsförderung dienenden Zubereitung, vorzugsweise als Protein- oder Aromastoffmischung. Der Verwendung kommen die für die Zubereitung beschriebenen Vorteile entsprechend zu.

Gemäß einer bevorzugten Ausführungsart wird die Zusammensetzung zur Herstellung eines Fleischersatzproduktes verwendet.

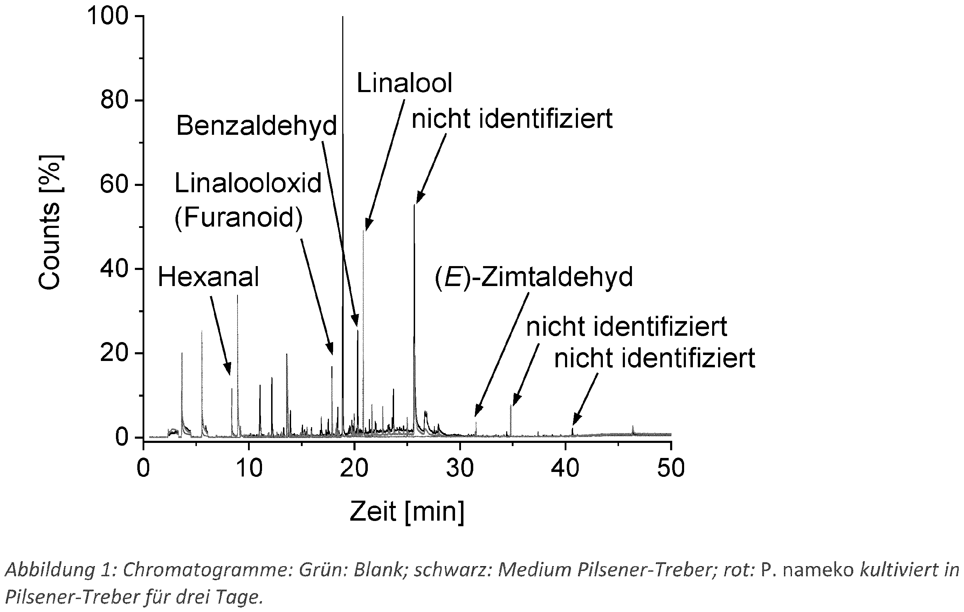

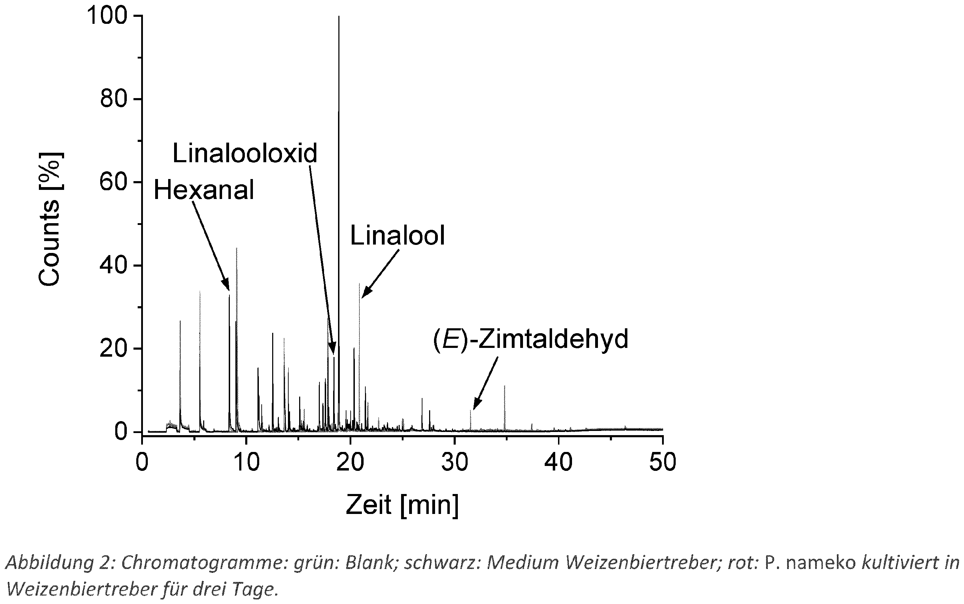

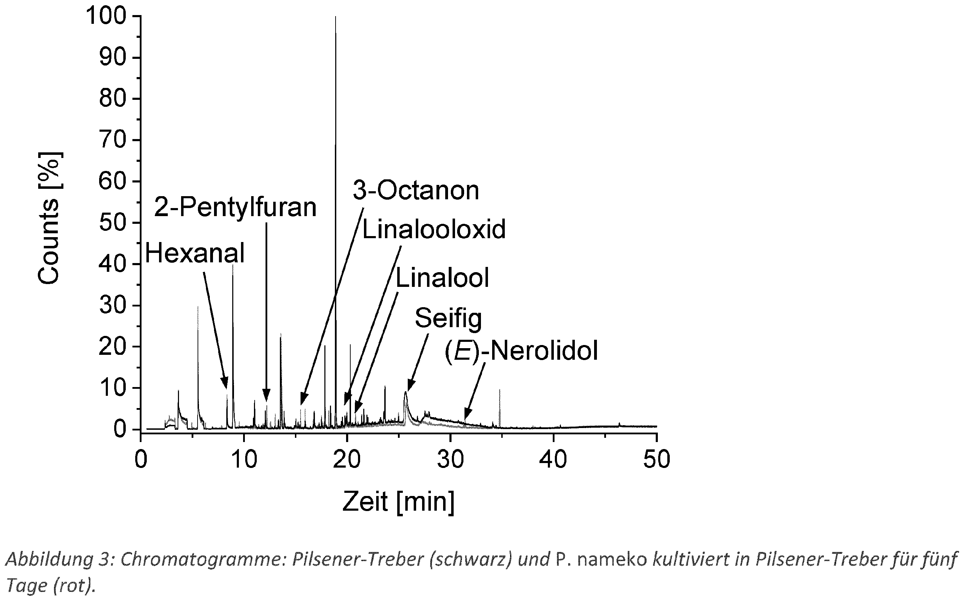

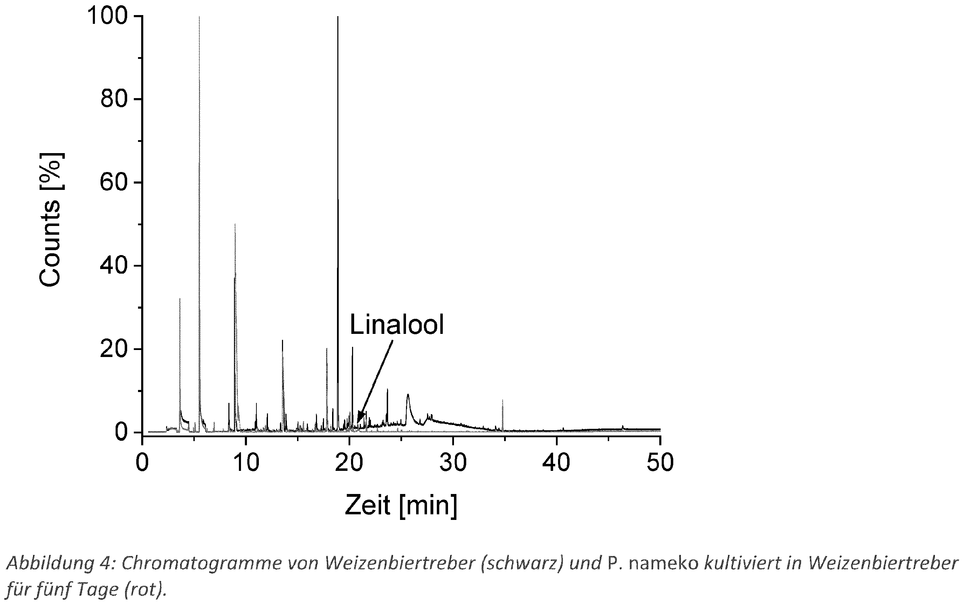

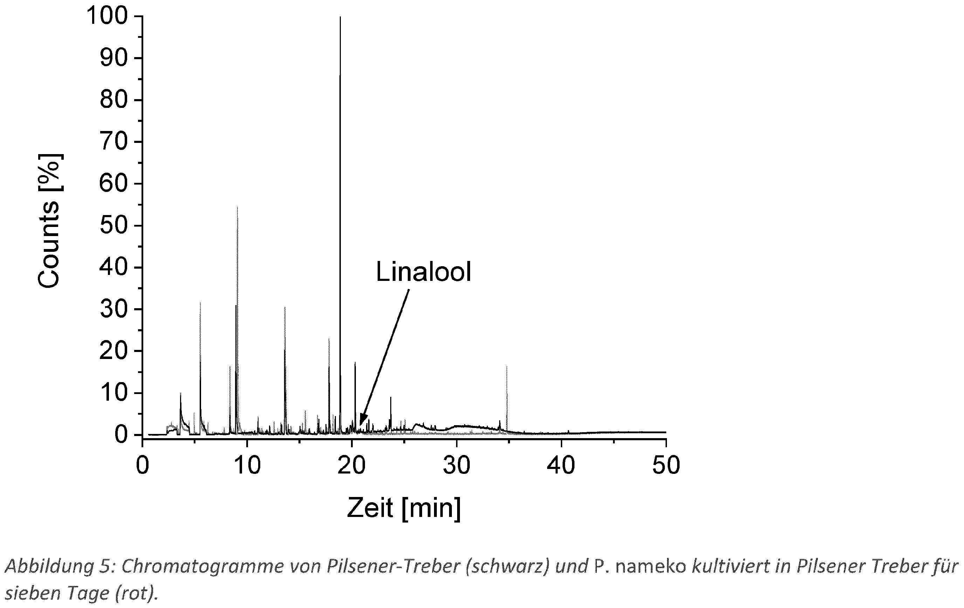

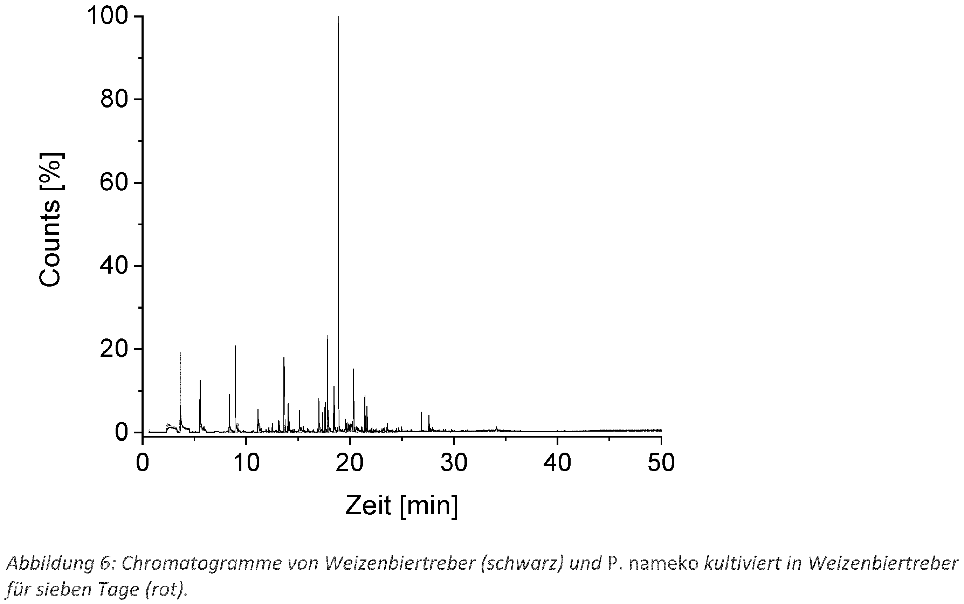

Nachfolgend wird die Erfindung anhand einer experimentellen Untersuchung mit ausgewählten Basidiomyceten unter Bezug auf die anliegenden Diagramme (Abb. 1 bis 6) näher erläutert.

Die Angaben zur Kultivierung (Pilzstämme, Stammhaltung, Vorkulturen, Hauptkulturen) können mit den entsprechenden Anpassungen für die Übertragung des Verfahrens vom Labormaßstab auf den technischen Maßstab verwendet werden.

### Material und Methoden

### Organismen

Die für die Arbeiten verwendeten Basidiomyceten sind in Tabelle 1 aufgeführt.

**Tabelle 1: Verwendete Basidiomyceten.**

| **Stamm** | **Trivialname** | **Abkürzung** | **Interne Stamm-Nr.** | **Herkunft** | **Stamm-Nr.** |
|---|---|---|---|---|---|
| *Pleurotus eryngii* | Brauner Kräuterseitling | PER | 100 | DSMZ | 8264 |
| *Pholiota nameko* | Japanisches Stockschwämmchen | PNA | 113 | DSMZ | 6908 |
| *Cyclocybe aegerita* | Südlicher Ackerling | AAE | 166 | Fa. Sylvan, Horst, NL | 4022 |

### Substrate

Die Treber von Weizen- und Pilsener Bier wurden von der Störtebeker Braumanufaktur zur Verfügung gestellt. Die Treber wurden im Thermomix^{®} homogenisiert und bis zur Verwendung bei -20 °C gelagert. Die Trockenmasse der Biertreber betrug ca. 20%, für die Medien wurde dieser feucht eingesetzt.

Der Lupinenklarlauf 1 sowie Lupinenklarlauf S4 wurden von der Firma PROLupin geliefert, gefroren, bei -20 °C gelagert und vor der Verwendung durch Schütteln homogenisiert. Der Trockenmassegehalt des Lupinenklarlaufs 1 betrug 2,8 g/L, der von S4 0,12 g/L.

### Kultivierung

Die Kultivierung erfolgte unter sterilen Bedingungen. Alle im Folgenden beschriebenen Medien wurden vor der Verwendung für 20 min bei 121 °C autoklaviert. Die Kulturführung fand unter Lichtausschluss bei 24 °C statt. Submerskulturen wurden bei 150 rpm geschüttelt.

### Stammhaltung

Die Stammhaltung der verwendeten Basidiomyceten erfolgte in Emerskulturen auf Malzextrakt-Agar-Platten (MEA; 20 g/L Malzextrakt und 15 g/L Agar-Agar in vollentsalztem Wasser (VE-Wasser)). Es wurden jeweils 0,25 cm² frisches Mycel aus einer zu 80% bewachsenen Agarplatte ausgestochen und auf eine neue Platte transferiert. Dieser Vorgang wurde jeweils bei einem Bewuchs von 80% der Agarplatten wiederholt.

### Vorkulturen

Die Vorkulturen wurden in Malzextraktmedium angesetzt. Als Medium diente 20 g/L Malzextrakt in VE-Wasser. Die Kulturen wurden in Erlenmeyerkolben kultiviert, welche zu 40% ihres Volumens mit flüssigem Medium gefüllt wurden. Es wurden 0,25 cm² frisches Mycel einer zu 80% bewachsenen Agarplatte ausgestochen, in das Medium überführt und mittels Ultra-Turrax Dispergiergerät für 30 s homogenisiert. Die Vorkultur wurde für sieben Tage kultiviert.

### Hauptkulturen

Die Hauptkulturen wurden ebenfalls in Erlenmyerkolben mit 40Vol.-% Medium gezüchtet. Die Zusammensetzung der *Fed-Batch* Medien für *P. eryngii* ist in Tabelle 2 aufgeführt. Für diese Medien wurde Weizenbiertreber in den angegebenen Mengen verwendet und das Medium anschließend mit Lupinenklarlauf 1 *ad* 40 Vol.-% aufgefüllt. Die Trockenmasse des Lupinenklarlaufs wurde hierbei nicht beachtet.

**Tabelle 2: Zusammensetzung der Fed-Batch Medien für die Kultivierung von P. eryngii.**

| **Kulturtag** | **Ansatz 1** | **Ansatz 2** | **Ansatz 3** | **Ansatz 4** |
|---|---|---|---|---|
| 0 | 10 g/L Trockenmasse | 10 g/L Trockenmasse | 5 g/L Trockenmasse | 5 g/L Trockenmasse |
| 2 | 10 g/L Trockenmasse | - | 5 g/L Trockenmasse | - |
| 4 | - | 10 g/L Trockenmasse | 5 g/L Trockenmasse | 5 g/L Trockenmasse |
| 7 | Ernte | Ernte | Ernte | Ernte |
| Insgesamt eingesetzte Biotrockenmasse Treber [g/L] | 20 | 20 | 15 | 10 |

Für die Kultivierung von *P. nameko* in Weizenbier- und Pilsener-Treber-Medien wurden jeweils 20 g/L Trockenmasse der homogenisierten Treber eingesetzt und mit Trinkwasser *ad* 40 Vol.-% im Kolben aufgefüllt.

*C. aegerita* wurde in 20 g Trockenmasse Pilsener-Treber pro Liter mit Lupinenklarlauf S4 *ad* 40 Vol.-% des Kolbens kultiviert und nach einer Woche geerntet.

Die Inokulation der Hauptkulturmedien erfolgte nach zweimaligem Waschen der homogenisierten Vorkulturen. Die Homogenisierung erfolgte analog zur Inokulation der Vorkulturen mittels Ultra-Turrax Dispergiergerät. Die Vorkulturen von *P. eryngii* wurden mit VE-Wasser gewaschen, die von *P. nameko* mit Trinkwasser.

### Ernte des Mycels

Nach Ende der jeweiligen Kultivierungsdauer wurde der Inhalt der Erlenmeyerkolben in ausgewogene Zentrifugenröhrchen überführt und für 10 min bei 3.500 g zentrifugiert. Der Überstand wurde verworfen und das Zentrifugenröhrchen erneut gewogen um die Biofeuchtmasse zu bestimmen. Anschließend wurden die Fermentate gefriergetrocknet. Die Zentrifugenröhrchen wurden erneut gewogen, um die Biotrockenmasse zu ermitteln. Für die weiteren Analysen wurden die Mycelien mittels RETSCH-Kugelmühle gemahlen und zu Pulver weiterverarbeitet.

### Analytik

### Bestimmung der Biomasse

Die Biotrockenmasse der Kulturen wurde aus der Differenz des getrockneten Mycels im Zentrifugenröhrchen und dem leeren Zentrifugenröhrchen gebildet.

### Bestimmung der Restfeuchte

Das gefriergetrocknete Mycel wurde mittels IR-Feuchtebestimmer analysiert, um die Restfeuchte zu bestimmen. In der Regel wurde die Bestimmung nach Herstellerangaben im Duplikat durchgeführt, wenn nicht genug Mycel vorhanden war, wurde eine Einfachbestimmung durchgeführt.

### Glutenanalyse

Für die Glutenanalysen wurden je eine Probe des Pilsener-Trebers und des Fermentats von Kulturtag sieben von *C*. *aegerita* gefriergetrocknet und mittels RETSCH Kugelmühle homogenisiert. Die Gluten-Analytik erfolgte mittels eines kompetitiven ELISA-Kits. Die Probe des Trebers wurde 1:10.000, die des Mycels 1:1.000 verdünnt.

### Bestimmung des Aminosäureprofils

Die Hydrolyse der Proben zur Aminosäureanalytik erfolgte auf drei unterschiedliche Weisen. Zum einen mittels saurer Totalhydrolyse, bei der Tryptohan, Cystein und Methionin nicht erfasst werden. Für die Bestimmung von Cystein und Methionin wurde ein oxidativer Aufschluss durchgeführt, damit diese als Cysteinsäure und Methioninsulfon bzw. Methioninsulfoxid analysiert werden können. Für die Bestimmung von Tryptophan wurde eine basische Hydrolyse durchgeführt.

### Bestimmung von Cystein und Methionin nach Oxidation

Zu Beginn wurde die Oxidationslösung für eine Stunde bei Raumtemperatur und anschließend für 15 min im Eisbad inkubiert. Von der Oxidationslösung wurden pro Probe 0,5 mL benötigt, welche sich aus 0,05 mL Wasserstoffperoxid (wt=30%) und 0,45 mL Ameisensäure (889 g Ameisensäure, 111 mL VE-Wasser und 4,73 g Phenol) zusammensetzt. Die Proben wurden in ein Zentrifugenröhrchen eingewogen und im Eisbad gekühlt. Je 0,5 mL der Oxidationslösung wurden zu den Proben gegeben und diese anschließend für 16 h im Eisbad bei 4 °C inkubiert.

Die Oxidation wurde durch die Zugabe von 84 mg Natriumdisulfit gestoppt. Anschließend wurden 2,5 mL phenolhaltige Salzsäure (6 M, 0,1% Phenol) hinzugegeben und die Proben wurden weitere 24 h inkubiert.

### Bestimmung der Gesamtaminosäuren nach saurer Hydrolyse

Die Proben wurden in Zentrifugenröhrchen eingewogen und mit 2,5 mL Salzsäure (6 M, 0,1% Phenol) versetzt. Die Inkubation erfolgte analog zur Bestimmung von Cystein und Methionin nach der Oxidation. Die Hydrolyse wurde durch Zugabe von Natronlauge (7,5 M) gestoppt und der pH-Wert auf 2,2 eingestellt. Anschließend wurden die Proben *ad* 20 mL mit dem Citratpuffer aufgefüllt, filtriert und vermessen.

### Bestimmung von Tryptophan nach alkalischer Hydrolyse

Die Proben wurden in Zentrifugenröhrchen eingewogen und mit 2,5 mL phenolischer Natronlauge (5 M, 0,1% Phenol) versetzt. Die Inkubation erfolgte analog zur sauren Hydrolyse bei 110 °C für 24 h, davon die erste Stunde mit leicht geöffnetem Deckel und 23 h mit fest verschlossenem Deckel. Nach dem Abkühlen der Proben wurde 1 mL Phosphorsäure (0,5 M) hinzugefügt, der pH-Wert auf 2,2 eingestellt und die Proben *ad* 20 mL in Messkolben mit Citratpuffer aufgefüllt. Vor der Analyse wurden die Proben membranfiltriert.

### Aminosäureanalysator

Die freigesetzten Aminosäuren wurden an einer Kationenaustauschersäule getrennt und mittels Nachsäulenderivatisierung mit Ninhydrin bei 570 nm beziehungsweise 440 nm für Prolin detektiert (Tabelle 3 - 4). Die Analyse erfolgt mittels Aminosäureanalysator S433 (SYKAM Vertriebs GmbH). Die Kalibration erfolgte über eine Einpunktkalibration.

**Tabelle 3: Geräteparameter der Aminosäureanalytik.**

| **Parameter** | | | | | | |
|---|---|---|---|---|---|---|
| Trennsäure | Kationentrennsäule LCA K13/Na für das erweiterte Hydrolysatprogramm; 4,6 x 175 mm | | | | | |
| Vorsäule | Ammoniakfiltersäule LCA K13/Na für Protein-Hydrolysat; 4,6 x 100 mm | | | | | |
| Eluenten | A: Natrium-Citratpuffer; 0,12 N; pH 3,45 (SYKAM) | | | | | |
| | B: Natrium-Citratpuffer; 0,20 N; pH 10,85 (SYKAM) | | | | | |
| Flussrate | D: Regenerationslösung: Natronlauge; 0,5 M mit 0,68 mM EDTA | | | | | |
| | 0,45 mL/min | | | | | |
| Aminomodul | Ninhydrin; 0,1 M in Methanol | | | | | |
| Flussrate | Waschlösung: Isopropanol/Ethanol/dd Wasser (1/1/2) 0,25 mL/min | | | | | |
| Gradient | **Zeit [min]** | **A[%]** | **B[%]** | **D[%]** | **Ninhydrin** | **Waschlsg.** |
| | 0 | 100 | 0 | 0 | 100 | 0 |
| | 11 | 95 | 5 | 0 | 100 | 0 |
| | 13 | 80 | 20 | 0 | 100 | 0 |
| | 25 | 70 | 30 | 0 | 100 | 0 |
| | 29 | 30 | 70 | 0 | 100 | 0 |
| | 31 | 20 | 80 | 0 | 100 | 0 |
| | 33 | 10 | 90 | 0 | 100 | 0 |
| | 41 | 0 | 100 | 0 | 100 | 0 |
| | 49,1 | 0 | 0 | 100 | 100 | 0 |
| | 50 | 0 | 0 | 100 | 0 | 100 |
| | 52,1 | 100 | 0 | 0 | 0 | 100 |
| | 65 | 100 | 0 | 0 | 100 | 0 |
| Säulenofen | **Zeit [min]** | | | **Temperatur [°C]** | | |
| | 0 | | | 49 | | |
| | 5 | | | 49 | | |
| | 10 | | | 56 | | |
| | 23 | | | 25 | | |
| | 28 | | | 74 | | |
| | 34 | | | 74 | | |
| | 39 | | | 50 | | |
| | 65 | | | 50 | | |
| Reaktortemperatur | 130 °C | | | | | |
| Injektionsvolumen | 50 µL | | | | | |
| Detektor | UV/Vis bei 570 nm und 440 nm (Prolin) | | | | | |
| Software | Chromstar 7.14 | | | | | |

**Tabelle 4: Geräteparameter der Tryptophananalyse.**

| **Parameter** | | | | | | |
|---|---|---|---|---|---|---|
| Eluenten | A: Natrium-Citratpuffer; 0,12 N; pH 3,45 (SYKAM) | | | | | |
| | B: Natrium-Citratpuffer; 0,20 N; pH 10,85 (SYKAM) | | | | | |
| | D: Regenerationslösung: Natronlauge; 0,5 M mit 0,68 mM | | | | | |
| Flussrate | EDTA | | | | | |
| | 0,45 mL/min | | | | | |
| Aminomodul | Ninhydrin; 0,1 M in Methanol | | | | | |
| | Waschlösung: Isopropanol/Ethanol/dd Wasser (1/1/2) | | | | | |
| Flussrate | 0,25 mL/min | | | | | |
| Gradient | **Zeit [min]** | **A[%]** | **B[%]** | **D[%]** | **Ninhydrin** | **Waschlsg.** |
| | 0 | 30 | 70 | 0 | 100 | 0 |
| | 7 | 10 | 90 | 0 | 100 | 0 |
| | 15 | 0 | 100 | 0 | 100 | 0 |
| | 20,1 | 0 | 0 | 0 | 100 | 0 |
| | 21 | 0 | 0 | 0 | 0 | 100 |
| | 23,1 | 30 | 70 | 0 | 0 | 100 |
| | 32 | 30 | 70 | 0 | 100 | 0 |
| Säulenofen | 74 °C | | | | | |
| Reaktortemperatur | 130 °C | | | | | |
| Injektionsvolumen | 50 µL | | | | | |
| Detektor | UV/Vis bei 570 nm | | | | | |
| Software | Chromstar 7.14 | | | | | |

### Gaschromatographie

Für die Bestimmung des Aromaprofils der Kulturen von *P. nameko* in Weizen- und Pilsener-Treber wurde an den jeweiligen Kulturtagen ein Aliquot von 4mL des Kulturüberstandes steril in eine 20 mL Vial überführt, mittels *Solid Phase Microextraction* (SPME) extrahiert und mittels Gaschromatographie-Massenspektrometrie-Olfaktometrie (GC-MS-O) vermessen. Für die Aromaprofile der Referenz-Medien wurde ein Erlenmeyerkolben mit sterilem Medium parallel inkubiert und ebenfalls an den jeweiligen Kulturtagen aliquotiert. Die Bedingungen der gaschromatographischen Analyse sind in Tabelle zusammengefasst.

**Tabelle 5: Geräteparameter der SPME-GC-MS-O Analytik.**

| **Parameter** | | |
|---|---|---|
| GC | Agilent Technologies 7890B GC System mit 5977B MSD (Agilent Technologies, Waldbronn, Deutschland); | |
| | Gerstel MPS Robotic (Gerstel GmbH & Co. KG, Mülheim a.d.R., Deutschland) | |
| **Extraktion** | | |
| SPME-Faser | Divinylbenzol (DVB) / Carboxen (CAR) / Polydimethylsiloxan (PDMS) | |
| Agitator | Inkubation: 10 min; 40 °C | |
| | | Agitator an: 30 s |
| | | Agitator aus: 3 s |
| | | Agitator Geschwindigkeit: 250 rpm |
| | Extraktion: 30 min; 40 °C | |
| | Desorption: 300 s | |
| **Injektion** | | |
| Liner | SPME-Liner / Glasverdampfrohr | |
| Modus | *splitless* | |
| **Trennung** | | |
| Trennsäule | VF-WAXms; 30 m x 250 µm x 0,25 µm | |
| Initiationstemperatur | 40 °C für 3 min | |
| Temperaturrampe | 5 °C min⁻¹ | |
| Temperaturmaximum | 240 °C für 7 min | |
| Laufzeit | 50 min | |
| Trägergas | He | |
| Fluss | 1,56 mL min⁻¹ | |
| **MS - Single Quadrupole** | | |
| Modus | *scan* (*m*/*z* 30-300) | |
| Ionisation | 70 eV | |
| Temperatur | lonenquelle: 230 °C | |
| Solvent-delay | 0 min | |
| **Olfaktometrie Detektor Port (ODP)** | | |
| Transferline | 250 °C | |
| Mischkammerheizung | 150 °C | |
| Makeup Gas | N₂ | |
| **Software** | | |
| Datenaufnahme | MassHunter GC/MS Aquisition B 07.05.2479 | |

| Maestro | Maestro 1 Version 1.5.3.81/3.5 | |
|---|---|---|
| Datenauswertung | MassHunter Workstation Qualitative Analysis Version B.07.00 | |
| Datenbank | NIST MS Search 2.2 | |

### Ergebnisse

### Biotrockenmassen

Die Biotrockenmassen der Kulturen von *P. eryngii* in den unterschiedlichen *Fed Batch* Medien (vgl. Tabelle 2) nach sieben Tagen sind in Tabelle 6 aufgeführt.

**Tabelle 6: Biotrockenmassen der Fed-Batch Kulturen von P. eryngii in Weizenbiertreber und Lupinenklarlauf.**

| | **Ansatz 1** | **Ansatz 2** | **Ansatz 3** | **Ansatz 4** |
|---|---|---|---|---|
| Biotrockenmasse [g/L] | 28,19 (± 0,615) | 27,37 (± 0,197) | 22,09 (± 0,206) | 19,22 (± 0,188) |

*C. aegerita* produzierte in 20 g/L Pilsener-Treber und Lupinenklarlauf S4 nach sieben Kulturtagen eine Trockenmasse von 18,88 ± 1,67 g/L.

Die Biotrockenmassen der kinetischen Reihe von *P. nameko* sind in Tabelle 7 gezeigt.

**Tabelle 7: Biotrockenmasse der kinetischen Reihe von P. nameko in Weizenbier- und Pilsener-Treber**

| **Kulturtag** | **Biotrockenmasse *P. nameko* in Pilsener-Treber [g/L]** | **Biotrockenmasse *P. nameko* in Weizenbiertreber [g/L]** |
|---|---|---|
| 3 | 22,73 (± 0,25) | 19,53 (± 1,3) |
| 5 | 21,92 (± 0,10) | 17,59 (± 0,44) |
| 7 | 20,44 (± 0,082) | 17,10 (± 0,22) |

### Glutenanalytik

Der Gliadingehalt des Pilsener-Trebers betrug 20,93 g/100 g. Der Gliadingehalt des fermentierten Trebers wurde zu 0,294 g/100 g bestimmt. Dies entspricht einem Glutenabbau von > 98%.

### Aminosäureprofil von P. eryngii kultiviert in Lupinenklarlauf und Weizenbiertreber

Die Aminosäureprofile der Kulturen von *P. eryngii* sind in Tabelle 8 aufgeführt. Die Angaben sind in g/100 g Trockenmasse Probe dargestellt und die Masse bezieht sich jeweils auf den *Aminoacidresidual,* also den Wert, den man erhält, wenn man berücksichtigt, dass die Aminosäuren im Protein gebunden vorliegen und bei deren Bindung ein Wassermolekül abgespalten wird. Die Ansätze beziehen sich auf die Medienzusammensetzung (vgl. Tabelle 2) und die zusätzliche Beschriftung A und B bezieht sich auf eine biologische Doppelbestimmung.

**Tabelle 8: Aminosäureresidual [g/100 g DM], in biologischem Duplikat kultiviert (A und B) und in Vierfachbestimmung analysiert**

| | **Ansatz 1 A** | **Ansat z 1 B** | **Ansat z 2 A** | **Ansat z 2 B** | **Ansat z 3 A** | **Ansatz 3 B** | **Ansatz 4 A** | **Ansat z 4 B** | **Weizenbiertreb er** |
|---|---|---|---|---|---|---|---|---|---|
| ALA | 1,527 (± 0,181) | 1,408 (± 0,029 ) | 1,386 (± 0,030 ) | 1,379 (± 0,004 ) | 1,257 (± 0,070 ) | 1,400 (± 0,037) | 1,204 (± 0,019) | 1,318 (± 0,042 ) | 0,991 (± 0,095) |
| ARG | 2,360 (± 0,337) | 2,070 (± 0,050 ) | 2,014 (± 0,039 ) | 1,917 (± 0,016 ) | 1,615 (± 0,091 ) | 2,208 (±0,03 5) | 1,493 (± 0,015) | 1,682 (± 0,79) | 1,169 (± 0,051) |
| ASP^{∗} | 2,648 (± 0,337) | 2,374 (± 0,045 ) | 2,317 (± 0,053 ) | 2,391 (± 0,006 ) | 2,068 (± 0,117 ) | 2,436 (± 0,056) | 1,935 (± 0,024) | 2,209 (± 0,075 ) | 1,554 (± 0,118) |
| CYS | 0,453 (± 0,009) | 0,486 (± 0,009 ) | 0,480 (± 0,031 ) | 0,458 (± 0,016 ) | 0,406 (± 0,007 ) | 0,477 (± 0,013) | 0,380 (± 0,005) | 0,394 (± 0,011 ) | 0,052 (± 0,002) |
| GLX^{∗} | 4,425 (± 0,553) | 4,286 (± 0,074 ) | 3,787 (± 0,072 ) | 4,069 (± 0,016 ) | 3,642 (± 0,208 ) | 4,189 (± 0,108) | 3,513 (± 0,034) | 3,917 (± 0,131 ) | 3,705 (± 0,354) |
| GLY | 1,235 (± 0,138) | 1,157 (± 0,020 ) | 1,097 (± 0,024 ) | 1,126 (± 0,004 ) | 0,993 (± 0,051 ) | 1,161 (± 0,028) | 0,929 (± 0,015) | 1,037 (± 0,034 ) | 0,784 (± 0,069) |
| HIS | 1,320 (± 0,196) | 1,181 (± 0,039 ) | 1,155 (± 0,060 ) | 0,981 (± 0,022 ) | 1,175 (± 0,089 ) | 1,228 (± 0,008) | 1,096 (± 0,039) | 1,164 (± 0,017 ) | 0,617 (± 0,013) |
| ILE | 1,212 (± 0,144) | 1,088 (± 0,030 ) | 1,080 (± 0,019 ) | 1,139 (± 0,010 ) | 0,919 (± 0,059 ) | 1,113 (± 0,039) | 0,856 (± 0,015) | 0,981 (± 0,038 ) | 0,710 (± 0,054) |
| LEU | 2,166 (± 0,276) | 1,932 (± 0,044 ) | 1,915 (± 0,041 ) | 1,972 (± 0,005 ) | 1,616 (± 0,091 ) | 1,972 (± 0,046) | 1,492 (± 0,032) | 1,686 (± 0,059 ) | 1,405 (± 0,060) |
| LYS | 1,426 (± 0,200) | 1,278 (± 0,023 ) | 1,220 (± 0,025 ) | 1,191 (± 0,005 ) | 0,992 (± 0,067 ) | 1,228 (± 0,028) | 0,918 (± 0,014) | 1,049 (± 0,038 ) | 0,869 (±0,008) |
| MET | 0,365 (±0,01 4) | 0,366 (± 0,006 ) | 0,398 (± 0,017 ) | 0,410 (± 0,016 ) | 0,313 (± 0,005 ) | 0,402 (± 0,018) | 0,282 (± 0,017) | 0,320 (± 0,014 ) | 0,368 (± 0,097) |
| PHE | 1,415 (± 0,195) | 1,263 (± 0,031 ) | 1,229 (± 0,029 ) | 1,257 (± 0,012 ) | 1,060 (± 0,059 ) | 1,288 (± 0,029) | 0,808 (± 0,248) | 1,095 (± 0,038 ) | 0,972 (± 0,011) |
| PRO | 1,368 (± 0,163) | 1,273 (± 0,029 ) | 1,228 (± 0,029 ) | 1,277 (± 0,010 ) | 1,083 (± 0,065 ) | 1,298 (± 0,035) | 0,947 (± 0,018) | 1,041 (± 0,035 ) | 1,626 (± 0,272) |
| SER | 1,392 (± 0,163) | 1,264 (± 0,024 ) | 1,199 (± 0,023 ) | 1,248 (± 0,006 ) | 1,155 (± 0,064 ) | 1,283 (± 0,029) | 1,106 (± 0,014) | 1,237 (± 0,037 ) | 0,839 (± 0,068) |
| THR | 1,337 (± 0,167) | 1,167 (± 0,022 ) | 1,154 (± 0,026 ) | 1,239 (± 0,004 ) | 1,097 (± 0,063 ) | 1,271 (± 0,028) | 1,014 (± 0,012) | 1,132 (± 0,038 ) | 0,633 (± 0,056) |
| TRP | 0,132 (± 0,006) | 0,128 (± 0,013 ) | 0,135 (± 0,001 ) | 0,127 (± 0,005 ) | 0,061 (± 0,008 ) | 0,091 (± 0,0005) | 0,078 (±0,00 6) | 0,071 (± 0,005 ) | 0,149 (± 0,003) |
| TYR | 0,678 (±0,10 2) | 0,566 (± 0,023 ) | 0,542 (± 0,010 ) | 0,615 (± 0,020 ) | 0,524 (± 0,028 ) | 0,656( ± 0,004) | 0,531 (± 0,016) | 0,593 (± 0,037 ) | 0,562 (± 0,020) |
| VAL | 1,556 (± 0,191 | 1,348 (± 0,027 ) | 1,360 (± 0,036 ) | 1,413 (± 0,009 ) | 1,140 (± 0,067 ) | 1,401 (± 0,033) | 1,072 (± 0,023) | 1,206 (± 0,047 ) | 1,007 (± 0,050) |
| **SUM** | **27,02 (± 3,34)** | **24,64 (± 0,47)** | **23,70 (± 0,47)** | **24,21 (± 0,08)** | **21,12 (± 1,17)** | **25,10 (± 0,56)** | **19,65 (± 0,42)** | **22,13 (± 0,71)** | **18,01 (± 0,61)** |
| **EAAI** | **99** | **99** | **99** | **99** | **98** | **98** | **97** | **98** | **88** |
| **BV** | **97** | **96** | **96** | **96** | **95** | **95** | **94** | **95** | **84** |
| **Limit .AA** | **LYS** | **LYS** | **LYS, CYS & MET** | **LYS** | **LYS** | **LYS, CYS & MET** | **LYS, CYS & MET** | **LYS** | **CYS & MET, TRP** |

| | | | | | | | | | |
|---|---|---|---|---|---|---|---|---|---|
| SUM: Summe EAAI: *essential Amino Acid Index* BV: *Biological Value* Limit. AA: Limitierende Aminosäure | | | | | | | | | |

Im Durchschnitt wurde für die Mycelien ein EAAI von 98,4 sowie eine biologische Wertigkeit von 95,5 bestimmt. Damit wurde die Wertigkeit des Proteins im Vergleich zum nicht fermentierten Treber (EAAI 88 bzw. BV 84) signifikant verbessert.

### Aromaanalytik

Die Kulturen von *P. nameko* wurden ebenfalls in biologischer Doppelbestimmung kultiviert und mittels SPME-GC-MS-O analysiert. Die Ergebnisse wahrgenommenen Geruchseindrücke zusammen mit Substanzvorschlägen sind in den Tabellen 9-14 aufgeführt, die zugehörigen Chromatogramme in den Abbildungen 1 - 6. Es sind jeweils die Chromatogramme des nicht inokulierten Mediums als Vergleich dargestellt.

**Tabelle 9: Aromaprofil von P. nameko kultiviert in Pilsener-Treber für drei Tage (Probe 1); RI: Retentionsindex.**

| **Retentionszeit [min]** | **RI** | **Substanzvorschlag (mit RI) NIST Datenbank** | **Geruchseindruck** | **RI Standard** |
|---|---|---|---|---|
| 5,42 | 953 | 2-Ethylfuran (950) | süßlich | |
| 8,38 | 1081 | Hexanal (1083) | pilzig, frisch | 1082 |
| 9,38 | 1118 | | süßlich | |
| 9,54 | 1124 | 2-Butylfuran (1123) | herb | |
| 13,07 | 1250 | 6-Methyl-3-heptanone (1247) | pilzig | |
| 13,41 | 1255 | | süßlich, pilzig | |
| 13,97 | 1282 | 2-Octanon (1287) | | 1279 |
| 14,46 | 1299 | 1-Octen-3-on (1300) | Champignon, frisch | 1301/1302 |
| 15,91 | 1353 | 1-Hexanol (1355) | würzig | 1349 |
| 17,20 | 1401 | | kautig | |
| 18,22 | 1442 | (Z)-Linalooloxid (1444) | erdig, waldig | 1442 |
| 19,89 | 1507 | 2-Acetylfuran (1499) | grün, seifig | |
| 20,33 | 1525 | Benzaldehyd (1520) | stechend, süßlich | 1519 |
| 20,84 | 1546 | Linalool (1547) | blumig, Blaubeere | 1549 |
| 23,35 | 1652 | Acetophenon (1647) | süßlich | |
| 24,19 | 1689 | | Champignon | |
| 24,65 | 1709 | (*E*)-Germacrene D (1710) | herb, süßlich | |
| 25,68 | 1756 | | pilzig | |
| 27,35 | 1834 | | angebrannt | |
| 31,30 | 2030 | | seifig | |
| 31,45 | 2038 | (*E*)-Nerolidol (2042) | säuerlich | 2042 |
| 31,52 | 2041 | (*E*)-Zimtaldehyd (2040) | | 2046 |
| 34,79 | 2218 | | Brühe, würzig, pilzig | |

**Tabelle 10: Aromaprofil von P. nameko kultiviert in Weizenbiertreber für drei Tage (Probe 2); RI: Retentionsindex.**

| **Retentionszeit [min]** | **RI** | **Substanzvorschlag (mit RI) NIST Datenbank** | **Geruchseindruck** | **RI Standard** |
|---|---|---|---|---|
| 4,74 | 918 | 2-Methylbutanal (918) | süßlich | 914 |
| 8,37 | 1081 | Hexanal (1083) | würzig | 1082 |
| 13,05 | 1249 | 1-Pentanol (1250) | leicht pilzig | 1251 |
| 14,58 | 1304 | 1-Octen-3-one (1300) | pilzig | 1301/1302 |
| 16,63 | | | pilzig | - |
| 17,58 | | | brenzlig | - |
| 17,95 | 1431 | 2-(*E*)-Octenal (1429) | herb | 1434 |
| 18,22 | 1441 | (Z)-Linalooloxid (1444) | grün | - |
| 18,71 | 1461 | (-)-*α*-Cubeben (1463) | herb, Kakao | - |
| 19,89 | 1507 | | seifig | - |
| 20,85 | 1547 | Linalool (1547) | blumig, süßlich | 1549 |
| 21,66 | 1580 | | grün | - |
| 27,40 | 1836 | Calamenen (1839) | Lakritz, würzig | - |
| 31,30 | 2030 | *γ*-Nonalacton (2024) | süßlich | - |
| 31,45 | 2038 | (*E*)-Nerolidol (2042) | frisch | 2042 |
| 31,52 | 2042 | (*E*)-Zimtaldehyd (2040) | | 2046 |
| 32,22 | 2078 | | säuerlich, muffig | - |
| 34,80 | 2219 | | stark pilzig, muffig | - |

**Tabelle 3: Aromaprofil von P. nameko kultiviert in Pilsener-Treber für fünf Tage (Probe 1); RI: Retentionsindex.**

| **Retentionszeit [min]** | **RI** | **Substanzvorschlag (mit RI) NIST Datenbank** | **Geruchseindruck** | **RI Standard** |
|---|---|---|---|---|
| 4,94 | 928 | | pilzig, Brühe | - |
| 6,26 | 997 | | leicht pilzig | - |
| 12,19 | 1219 | 2-Pentylfuran (1231) | seifig, holzig, erdig | - |
| 13,03 | 1248 | 3-Octanon (1253) | holzig, pilzig | 1249 |
| 13,99 | 1283 | Octanal (1289) | süßlich, fruchtig | 1284 |
| 14,49 | 1301 | 1-Octen-3-on (1303) | Champignon, frisch | 1301/1302 |
| 17,15 | 1400 | | pilzig | - |
| 18,21 | 1441 | (Z)-Linalooloxide (furanoid) (1444) | grün | - |
| 20,31 | 1524 | Benzaldehyd (1524) | seifig, stechend | 1519 |
| 20,81 | 1545 | Linalool (1547) | Blaubeere, blumig | 1549 |
| 23,26 | 1648 | 2-Acetylthiazol (1642) | brotig, muffig | 1643 |
| 24,62 | 1708 | | pilzig | - |
| 24,97 | 1724 | | seifig | - |
| 31,43 | 2037 | (*E*)-Nerolidol (2042) | nicht wahrgenommen | 2042 |

**Tabelle 42: Aromaprofil von P. nameko kultiviert in Weizenbiertreber für fünf Tage (Probe 2); RI: Retentionsindex.**

| **Retentionszeit [min]** | **RI** | **Substanzvorschlag (mit RI) NIST Datenbank** | **Geruchseindruck** | **RI Standard** |
|---|---|---|---|---|
| 4,93 | 927 | | pilzig, Brühe | - |
| 6,26 | 997 | | leicht pilzig | - |
| 7,08 | 1030 | | pilzig | - |
| 8,37 | 1081 | | Brühe, würzig | - |
| 12,93 | 1245 | | Brühe | - |
| 14,44 | 1299 | 1-Octen-3-on (1303) | pilzig, frisch | 1301/1302 |
| 15,55 | 1340 | | angebrannt, süßlich | - |
| 17,9 | kein Peak | | Kakao, süßlich | - |
| 18,2 | 1441 | (Z)-Linalooloxid (1444) | grün | - |
| 18,93 | 1469 | (Z)-Linalooloxid (1465) | frisch, Minze | - |
| 19,43 | 1489 | | Schokolade | - |
| 20,84 | 1546 | Linalool (1547) | seifig, Blaubeere | 1549 |
| 24,67 | 1710 | | pilzig, erdig | - |
| 25,03 | 1728 | *α*-Muurolen (1726) | erdig, waldig | - |
| 25,79 | 1761 | *δ*-Cardinen (1758) | grün, stechend | - |
| 29,19 | 1923 | | süßlich, würzig | - |
| 29,8 | 1953 | | krautig | - |
| 35,42 | 2254 | | pilzig, erdig, muffig | - |

**Tabelle 53: Aromaprofil von P. nameko kultiviert in Pilsener-Treber für sieben Tage (Probe 1); RI: Retentionsindex.**

| **Retentionszeit [min]** | **RI** | **Substanzvorschlag (mit RI) NIST Datenbank** | **Geruchseindruck** | **RI Standard** |
|---|---|---|---|---|
| 4,94 | 928 | | pilzig | - |
| 6,71 | 1016 | | frisch | - |
| 7,27 | 1038 | 2-Methyl-3-buten-2-ol (1038) | frisch, seifig | 1038 |
| 7,82 | 1059 | | würzig, Brühe | - |
| 8,24 | 1076 | | brotig | - |
| 8,38 | 1081 | Hexanal (1083) | grün, seifig | 1082 |
| 8,72 | 1095 | | seifig | - |
| 11,2 | 1183 | Heptanal (1184) | würzig, krautig | 1187 |
| 12,93 | 1245 | | Brühe | - |
| 13,57 | 1268 | | seifig | - |
| 14,4 | kein Peak | | pilzig, frisch | - |
| 15,29 | 1330 | | pilzig | - |
| 16,72 | 1384 | | herb, pfeffrig | - |
| 17,34 | 1407 | | pilzig | - |
| 18,94 | 1470 | (Z)-Linalooloxid (1465) | süßlich, stechend, fruchtig | - |
| 19,94 | 1509 | | seifig, frisch | - |
| 20,85 | 1547 | Linalool (1547) | blumig, seifig | 1549 |
| 21,12 | 1558 | | süßlich, fruchtig | - |
| 23,62 | 1649 | | herb, sojaartig | - |
| 24,27 | 1692 | *γ*-Muurolen (1692) | brotig | - |
| 24,68 | 1710 | | pilzig, brenzlig | - |
| 25,07 | 1728 | *α* Muurolen (1726) | Anis, würzig | - |
| 25,8 | 1762 | *δ*-Cadinen (1758) | grün, frisch | - |
| 27,0 | 1817 | Tridecanal (1823) | wachsig, süßlich | - |
| 31,45 | 2038 | | frisch | - |
| 3,97 | 2173 | | frisch, herb, Chlor | - |
| 34,79 | 2254 | | muffig, erdig | - |

**Tabelle 64: Aromaprofil von P. nameko kultiviert in Weizen-Treber für sieben Tage (Probe 2); RI: Retentionsindex.**

| **Retentionszeit [min]** | **RI** | **Substanzvorschlag (mit RI) NIST Datenbank** | **Geruchseindruck** | **RI Standard** |
|---|---|---|---|---|
| 4,95 | 928 | | pilzig, muffig | - |
| 6,26 | 997 | | süßlich, brotig | - |
| 8,39 | 1082 | Hexanal (1083) | fruchtig, süßlich | 1082 |
| 12,63 | 1234 | 2-Pentylfuran (1231) | herb, pilzig | - |
| 12,89 | 1244 | | muffig | - |
| 13,22 | 1255 | 3-Octanon (1253) | pilzig | 1249 |
| 14,46 | 1300 | 1-Octen-3-on (1303) | Champignon, frisch | 1301/1302 |
| 16,73 | 1384 | | herb | - |
| 17,61 | 1418 | | Kakao, herb | - |
| 18,94 | 1470 | (Z)-Linalooloxid (1465) | seifig, süßlich | - |
| 20,85 | 1546 | Linalool (1547) | seifig | 1549 |
| 22,12 | 1599 | 2-Undecanon (1598) | süßlich, sahnig | 1602 |
| 24,45 | 1700 | | würzig, brotig | - |
| 24,68 | 1711 | | erdig | - |
| 25,07 | 1728 | *α*-Muurolen (1726) | süßlich, seifig | - |
| 25,8 | 1762 | | seifig, grün | - |
| 30,07 | 1967 | | säuerlich, brotig | - |
| 31,52 | 2041 | (*E*)-Zimtaldehyd (2040) | süßlich, frisch | 2046 |
| 32,14 | 2074 | | würzig | - |
| 34,1 | 2179 | Nonansäure (2171) | Lösemittel, wachsig | - |
| 34,8 | 2218 | | würzig | - |
| 35,43 | 2254 | | krautig | - |

## Patentansprüche

1. Verfahren zum Herstellen von Proteinen umfassend die folgenden Schritte:
• Bereitstellen eines Nährmediums enthaltend einen Treber aus der Herstellung von Bier oder einem anderen Getränk auf der Basis von Getreide,
• Zusammenbringen des Nährmediums mit mindestens einem Pilz aus der Abteilung der Ständerpilze (Basidiomyceten), der imstande ist, auf dem Medium eine Mischung von Proteinen zu bilden,
• Umsetzen des Trebers zu der Mischung von Proteinen mithilfe des Pilzes.

2. Verfahren nach Anspruch 1, bei dem der Ständerpilz ausgewählt ist aus der nachfolgenden Gruppe von Pilzen: Pleurotus eryngii, Pholiota nameko, Cyclocybe aegerita.

3. Verfahren nach Anspruch 1 oder 2, bei dem die Mischung von Proteinen eine biologische Wertigkeit von mindestens 94, vorzugsweise mindestens 97 aufweist.

4. Verfahren nach einem der Ansprüche 1 bis 3, bei dem die Mischung von Proteinen mindestens einen Aromastoff oder Mischung von Aromastoffen enthält, der mindestens eine Verbindung enthält, die aus der Stoffklasse der Terpene (z.B. Linalool, Linaloooxid, (*E*)-Nerolidol), der Carbonyle (z.B. 1-Octen-3-on, Zimtaldehyd), der Lactone (z.B. gamma-Nonalacton) und der Alkohole (z.B. 1-Hexanol, 2-Methyl-3-buten-2-ol ausgewählt ist.

5. Verfahren nach einem der Ansprüche 1 bis 4, bei dem der Ständerpilz ausgewählt ist aus einer Gruppe von Ständerpilzen mit der Eigenschaft, den Glutenanteil des Nährmediums um mindestens 90% abzubauen.

6. Verfahren nach einem der Ansprüche 1 bis 5, bei dem die Mischung von Proteinen einen Gliadingehalt von maximal als 0,5 g/100 g aufweist.

7. Verfahren nach einem der Ansprüche 1 bis 6, bei dem das Nährmedium aus Biertreber besteht oder im Wesentlichen aus Biertreber besteht.

8. Verfahren nach einem der Ansprüche 1 bis 7, und/oder bei dem das Medium neben dem Biertreber einen weiteren Nebenstrom aus einem Bierbrauprozess umfasst.

9. Verfahren nach einem der Ansprüche 1 bis 8, bei dem die Umsetzung in einer Submers-Kultur durchgeführt wird.

10. Verfahren nach einem der Ansprüche 1 bis 9, bei dem die Mischung von Proteinen aus dem mittels der Pilze umgesetzten Medium abgetrennt wird.

11. Protein- und aromastoffreiche Zusammensetzung herstellbar nach dem Verfahren einer der vorhergehenden Ansprüche.

12. Zusammensetzung nach Anspruch 11, die eine biologische Wertigkeit von mindestens 94, vorzugsweise mindestens 97 aufweist.

13. Zusammensetzung nach Anspruch 10 oder 12, umfassend einen Aromastoff oder eine Mischung von Aromastoffen, die mindestens eine Verbindung enthält, die aus der Stoffklasse der Terpene (z.B. Linalool, Linaloooxid, (*E*)-Nerolidol), der Carbonyle (z.B. 1-Octen-3-on, Zimtaldehyd), der Lactone (z.B. gamma-Nonalacton) und der Alkohole (z.B. 1-Hexanol, 2-Methyl-3-buten-2-ol) ausgewählt ist.

14. Zusammensetzung nach einem der Ansprüche 11 bis 13, enthaltend maximal 0,5 g/100 g Gliadin.

15. Der Ernährung, dem Genuss oder der Gesundheitsförderung dienende Zubereitung umfassend oder bestehend aus einer Zusammensetzung gemäß einem der Ansprüche 11 bis 14.

16. Verwendung einer Zusammensetzung gemäß einem der Ansprüche 11 oder 12 als oder in einer der Ernährung, dem Genuss oder der Gesundheitsförderung dienenden Zubereitung, vorzugsweise als Protein- und Aromastoffmischung.

17. Verwendung nach Anspruch 16 zur Herstellung eines Fleischersatzproduktes.

18. Verwendung nach Anspruch 16 oder 17 zur Herstellung eines Nahrungsmittels, Nutraceuticals, Genussmittels oder Arzneimittels.

## Geänderte Patentansprüche

### Geänderte Patentansprüche gemäss Regel 137(2) EPÜ.

1. Verfahren zum Herstellen von Proteinen umfassend die folgenden Schritte:
• Bereitstellen eines Nährmediums enthaltend einen Treber aus der Herstellung von Bier oder einem anderen Getränk auf der Basis von Getreide,
• Zusammenbringen des Nährmediums mit mindestens einem Pilz aus der Abteilung der Ständerpilze (Basidiomyceten), der imstande ist, auf dem Medium eine Mischung von Proteinen zu bilden,
• Umsetzen des Trebers zu der Mischung von Proteinen mithilfe des Pilzes, **dadurch gekennzeichnet, dass**
• der Ständerpilz ausgewählt ist aus einer Gruppe von Ständerpilzen mit der Eigenschaft, den Glutenanteil des Nährmediums so abzubauen, dass er im fermentierten Nährmedium um mindestens 90% geringer als im Nährmedium vor dem Fermentieren ist.

2. Verfahren nach Anspruch 1, bei dem der Ständerpilz ausgewählt ist aus der nachfolgenden Gruppe von Pilzen: *Pleurotus eryngii*, *Pholiota nameko*, *Cyclocybe aegerita.*

3. Verfahren nach Anspruch 1 oder 2, bei dem die Mischung von Proteinen eine biologische Wertigkeit von mindestens 94 aufweist.

4. Verfahren nach einem der Ansprüche 1 bis 3, bei dem die Mischung von Proteinen mindestens einen Aromastoff oder Mischung von Aromastoffen enthält, der mindestens eine Verbindung enthält, die aus der Stoffklasse der Terpene, der Carbonyle, der Lactone und der Alkohole ausgewählt ist.

5. Verfahren nach einem der Ansprüche 1 bis 4, bei dem die Mischung von Proteinen einen Gliadingehalt von maximal als 0,5 g/100 g aufweist.

6. Verfahren nach einem der Ansprüche 1 bis 5, bei dem das Nährmedium aus Biertreber besteht oder im Wesentlichen aus Biertreber besteht.

7. Verfahren nach einem der Ansprüche 1 bis 6, bei dem das Nährmedium neben dem Biertreber einen weiteren Nebenstrom aus einem Bierbrauprozess umfasst.

8. Verfahren nach einem der Ansprüche 1 bis 7, bei dem die Umsetzung in einer Submers-Kultur durchgeführt wird.

9. Verfahren nach einem der Ansprüche 1 bis 8, bei dem die Mischung von Proteinen aus dem mittels der Pilze umgesetzten Medium abgetrennt wird.

10. Protein- und aromastoffreiche Zusammensetzung herstellbar nach dem Verfahren einer der vorhergehenden Ansprüche.

11. Zusammensetzung nach Anspruch 10, die eine biologische Wertigkeit von mindestens 94 aufweist.

12. Zusammensetzung nach Anspruch 10 oder 11, umfassend einen Aromastoff oder eine Mischung von Aromastoffen, die mindestens eine Verbindung enthält, die aus der Stoffklasse der Terpene, der Carbonyle, der Lactone und der Alkohole ausgewählt ist.

13. Zusammensetzung nach einem der Ansprüche 10 bis 12, enthaltend maximal 0,5 g/100 g Gliadin.

14. Der Ernährung, dem Genuss oder der Gesundheitsförderung dienende Zubereitung umfassend oder bestehend aus einer Zusammensetzung gemäß einem der Ansprüche 10 bis 13.

15. Verwendung einer Zusammensetzung gemäß einem der Ansprüche 10 bis 13 als oder in einer der Ernährung, dem Genuss oder der Gesundheitsförderung dienenden Zubereitung.

16. Verwendung nach Anspruch 15 zur Herstellung eines Fleischersatzproduktes, Nahrungsmittels, Nutraceuticals, Genussmittels oder Arzneimittels.
